# EUROPEAN PATENT SPECIFICATION

(11) **EP 0 615 764 B1**
(45) Date of publication and mention of the grant of the patent: **19.05.1999**
(21) Application number: 94301859.8
(22) Date of filing: 16.03.1994
(51) Int. Cl.: A61M 16/00

(54) **System and method for closed loop inspiratory pressure control in a ventilator**
Vorrichtung und Verfahren zur Steuerung eines geschlossenen Regelkreises zum Einatmungsdruck in einem Beatmungsgerät
Système et méthode pour le contrôle en bouche fermée de la pression inspiratoire d'un respirateur

(30) Priority: 16.03.1993 US 33259
(43) Date of publication of application: 21.09.1994
(73) Proprietor: PURITAN-BENNETT CORPORATION, Kansas City, Kansas (US)
(72) Inventor: Isaza, Fernando J., San Marcos, California 92069 (US); Wong, Stanley Y., Oceanside, California 92056 (US)
(74) Representative: Mayes, Stuart David

(56) References cited:
- EP-A- 0 402 951
- US-A- 4 527 557
- US-A- 4 838 257

## Description

### BACKGROUND OF THE INVENTION

### Field of the Invention:

This invention relates generally to breathing ventilators, and more particularly relates to a system and method for controlling patient airway pressure during the inspiratory cycle of a breath by control of a flow delivery valve and a relief valve in combination to achieve a desired pressure level.

### Description of Related Art:

Medical ventilators are designed to assist a patient in breathing when the patient is somehow unable to adequately breathe on his own. In some form of ventilator systems, the patient is provided with ventilation support in the form of pressure assistance after the patient has began his inspiratory effort. With such a system, it is desirable to immediately increase the pressure after a breath is initiated in order to reach a target pressure input by the care provider. This rise in pressure causes flow to be initiated in the patient airway which supplies breathing gas to the patient's lungs. Conventional pressure controlled ventilator systems control the flow of gas to the patient by use of a pressure controller, a flow controller and flow valve in the inlet side of the airway, terminating the flow when the target pressure is reached.

However, such a control strategy often results in over pressurization of the patient, with the pressure overshooting the target pressure by a greater or lesser amount depending on the physiology of the patient and the construction of the airway. Often, the overshoot is sustained, thus over pressurizing the patient's lungs for the entire inspiration cycle of the breath. When this occurs, the possibility exists that the patient will be harmed by the higher than desirable pressure in the lungs. For instance, if the patient has recently had thoracic or abdominal surgery, the possibility exists that such over pressures could rupture sutures or blood vessels recently repaired in the surgery. Similarly, a very frail or infirm patient such as one with asthma or emphysema could be harmed if the breath pressure is in excess of that desired.

For these reasons, it would be desirable if the delivery of breaths in a pressure controlled ventilator could be controlled to eliminate overshoot or long term over pressurization in the patient airway. It would be particularly advantageous if the control of such overshoots could be accomplished without significant changes to the basic configuration and utilized existing components.

One suggested solution to the problem of over pressuristation is described in US-A-4,527,557 to include the provision of an exhalation valve. However, as described, the exhalation valve operates simply as a safety valve, venting the system when an excess pressure is sensed. There is no suggestion of the exhalation valve being actively controlled. Nevertheless, US-A-4,527,557 does describe a medical ventilator system having the features set out in the pre-characterising portions of the accompanying claims.

### SUMMARY OF THE INVENTION

According to a first aspect of the present invention there is provided a method of controlling airway pressure in a breathing gas circuit of a ventilator for a patient during the inspiratory cycle of a breath, the breathing gas circuit having a source of breathing gas, an airway flow path in fluid communication with a breathing attachment of the patient for supplying the breathing gas to the breathing attachment, a flow supply valve for controlling the supply of breathing gas to the breathing attachment, and a relief valve for controlling venting of breathing gas from the patient to the atmosphere, the method comprising the step of delivering a supply flow of breathing gas to the breathing attachment through the flow supply valve at a predetermined rate of supply flow based upon a desired airway pressure and actual airway pressure, characterised in that the method further comprises the steps of determining a desired rate of outflow, measuring the actual rate of outflow through the relief valve, comparing the actual rate of outflow with the desired rate of outflow to determine an outflow error, generating a relief valve command signal derived from a signal representing the desired airway pressure and a signal representing the outflow error to control operation of the relief valve, and, during the inspiratory cycle, venting the breathing gas from the breathing attachment through the relief valve in accordance with the relief valve command signal to achieve the desired airway pressure.

By use of embodiments of the invention, improved control of the pressure in the patient airway is obtained, particularly with regard to controlling overshoot and sustained over-pressurisation during the inspiratory cycle.

A current problem encountered during the inspiratory cycle of patient respirators is the control of overshoot of patient airway pressure during the inspiration cycle for pressure controlled type of ventilation. When the cycle is primarily controlled by the standard pressure and flow controllers, the patient is likely to experience an overshoot in the airway pressure as the controllers command the flow valve opening to rapidly supply breathing gas to the patient during the initial phases of the inspriation. Such an overpressure is often unavoidable if rapid filling of the patient airway is required and only control of the flow valve is available. Thus, it is highly desirable to provide more accurate control of patient airway pressure during the inspiratory cycle of the breathing cycle, since over-pressurisation of the patient's lungs has a number of undesirable effects.

According to a second aspect of the present invention there is provided a system for closed loop control of airway pressure in a breathing gas circuit of a ventilator for a patient during the inspiratory cycle of a breath, the breathing gas circuit having a source of breathing gas, and an airway flow path in fluid communication with a breathing attachment of the patient for supplying the breathing gas to the breathing attachment, the system comprising flow supply valve means in said airway flow path for delivering a supply flow of breathing gas from the source of breathing gas to the breathing attachment at a desired rate of supply flow based upon a desired airway pressure and the actual airway pressure; and relief valve means for the venting of breathing gas from the breathing attachment to the atmosphere during the inspiratory cycle, characterised in that the system further includes means for determining a desired rate of outflow, means for measuring the actual rate of outflow through the relief valve means, means for comparing the actual rate of outflow with the desired rate of outflow to determine an outflow error, and means for generating a relief valve command signal derived from a signal representing the desired airway pressure and a signal representing the outflow error to control operation of the relief valve means to vent the breathing gas from the breathing attachment through the relief valve means during the inspiratory cycle to achieve the desired airway pressure.

Using the system described, the pressure in the patient airway can be controlled to avoid overshoot, even if very aggressive initial flow rates are used to insure rapid filling of the lungs during the first stages of the inspiration cycle.

According to a third aspect of the present invention there is provided an apparatus for the control of airway pressure in a ventilation respirator of a patient according to claim 7.

In a presently preferred embodiment of this aspect of the invention, the desired patient airway pressure is summed with the measured patient airway pressure to create an error signal to be used as an input to a pressure controller. The pressure controller outputs a flow control signal representing the desired flow to achieve the desired pressure. The flow control signal is summed with the output of the flow valve to provide an error signal used to drive the flow valve controller. A relief valve, which is preferably an adjustable, actively controlled valve, is connected to the patient airway and driven by a command signal derived from a signal representing a desired pressure and a relief flow error signal. The relief error signal is based on the difference between the measured relief valve flow and the desired relief valve flow, which may be established based on a number of factors related to system and medical parameters. For example, patients are often medicated by nebulizers and the like, which results in the presence of unabsorbed medicants in the exhaled breathing gas. In such a case it may be desirable to limit the relief valve flow to a relatively low level such as 1 Liter/minute in order to limit exposure of others to the patient's medication. In general, it is desirable to limit the relief valve flow to limit the usage of gasses by the respirator. In a presently preferred embodiment, the relief valve may advantageously be an exhalation valve of the force balance variety well known in the art. Such valves may be controlled by electrical, pneumatic or mechanical inputs.

From the above, it can be seen that embodiments of the present invention provide an improved method and apparatus for the control of patient airway pressure during the inspiratory cycle of the patient breath on a ventilator by actively controlling both the flow control valve and a relief valve, which can be an exhalation valve, thereby providing both a rapid rise time of pressure and flow and a reduction in overshoot of airway pressure past a desired target pressure. Other properties and advantages of the invention will be apparent from the following detailed description and the accompanying drawings, which illustrate, by way of example, the features of the invention.

### BRIEF DESCRIPTION OF THE DRAWINGS

Fig. 1 is a schematic diagram of a prior art ventilator pressure controlled inspiratory breath control system;
Fig. 2 is schematic diagram of an embodiment of the pressure controlled ventilator inspiratory breath control system of the invention;
Fig. 3a is an illustration of a balloon type force balance valve;
Fig. 3b is an illustration of a diaphragm-type force balance valve;
Fig. 4(a) is an illustration of patient pressure during a pressure controlled inspiration phase using a prior art controller;
Fig. 4(b) is an illustration of patient flow during a pressure controlled inspiration phase using a prior art controller;
Fig. 5(a) is an illustration of patient pressure during a pressure controlled inspiration phase using the control system of Figure 2; and
Fig. 5(b) is an illustration of patient flow during a pressure controlled inspiration phase using the control system of Figure 2.

### DETAILED DESCRIPTION OF PREFERRED EMBODIMENTS

Pressure controlled ventilation systems provide pressure supported flow of breathing gas to a patient after the ventilator has sensed the initiation of a breath by the patient. However, if the flow of gas is not precisely controlled, the patient airway, and thus the patient's lungs, can be over pressurized by a pressure rise above the target level. In such an event, the patient can experience discomfort or possible harm from the effect of the higher pressure on the respiratory system.

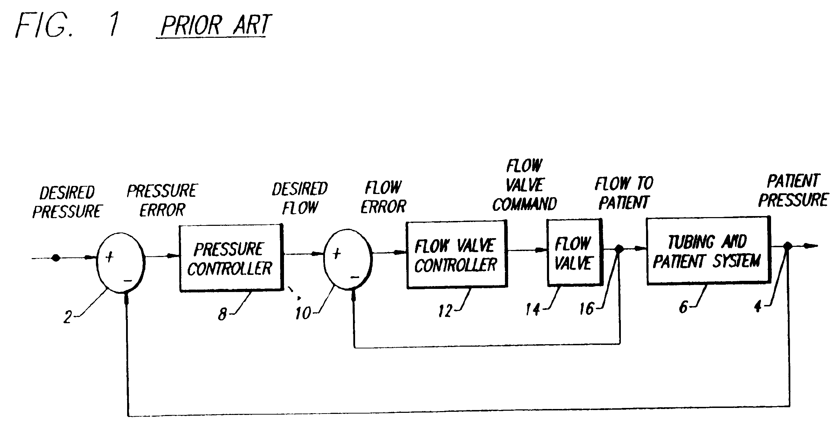

A prior art pressure controlled ventilator control system illustrated in Fig. 1 includes a means to input a desired pressure to an error summing means 2 which also receives an input from a pressure sensor 4 measuring patient pressure in the patient airway system 6. The difference between measured pressure and desired pressure is an error signal which is used as an input to the pressure controller 8 which generates a signal representing desired flow rate. The desired flow rate is summed with the signal from flow sensor 16 by summing means 10 to create a flow error signal used as an input to flow valve controller 12, which generates a flow valve command to drive flow valve 14. Flow valve 14 controls flow to the patient airway and tubing system 6.

Using such a system, the reservoir represented by the patient airway and the patient's lungs is filled rapidly after initiation of a breath with pressure support. Ideally, gas flow should be cut off as soon as a target pressure is reached; however, the dynamic response of prior art controllers and gas delivery systems often results in an overshoot of pressure resulting in over pressurization of the patient.

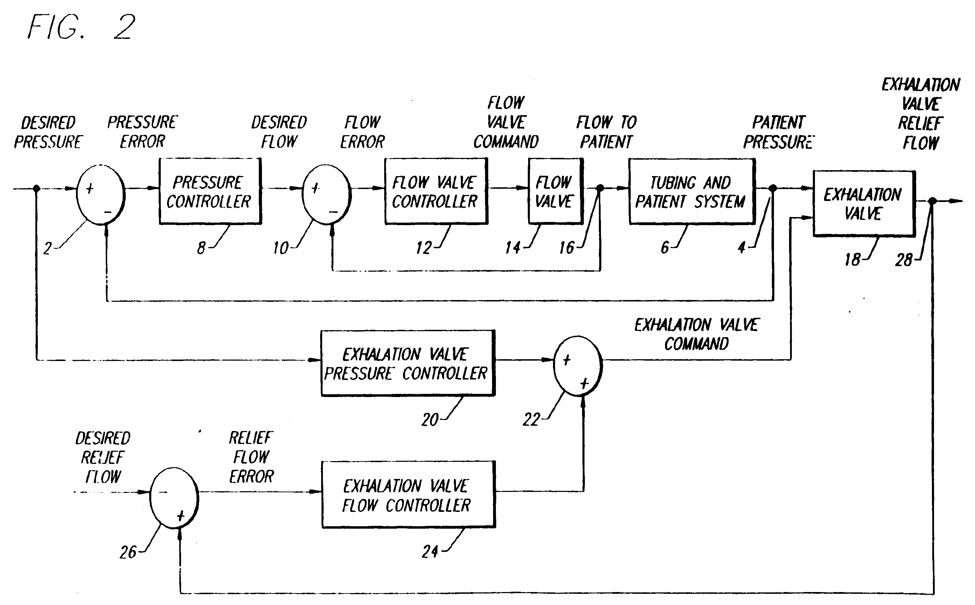

The present invention is embodied in a system and method for actively controlling both the inspiratory flow and pressure and a relief valve in the patient circuit system to prevent significant sustained overshoot of pressure beyond the target set by the care provider. As illustrated in Fig. 2, one embodiment of the present invention utilizes a pressure controlled ventilation system of the type illustrated in Fig. 1, along with active control of a relief valve, which could be the exhalation valve, to control the airway pressure. More specifically, summer 2 outputs an error signal which is used as an input to the pressure controller 8 on the basis of an input desired pressure and the measured airway pressure from pressure sensor 4. Pressure controller 8 outputs a desired flow rate which is summed by summing means 10 with the flow rate measured by flow sensor 16. The output of summing means 10 is used to drive flow valve controller 12 and thus flow valve 14 to provide a rate of flow to the patient through patient airway 6. The embodiment also inputs the desired pressure to an exhalation valve controller 20, the output of which is summed by summing means 22 to create an exhalation valve command to drive the exhalation valve 18. The other input to summing means 22 is the output of exhalation valve flow controller 24, which is in turn driven by the output of summing means 26. Summing means 26 outputs a relief flow error signal input to exhalation valve flow controller derived from a desired relief flow and the exhalation valve relief flow measured by exhalation valve relief flow sensor 28.

By use of the embodiment, overshoot of pressure in the patient airway is actively controlled by the exhalation valve on the basis of sensed flow and desired pressure. Another benefit of the embodiment is that a desired pressure trajectory may also be input and the trajectory will be accurately tracked using the closed loop system. Thus, a variety of respiration strategies other than mere fixed level target pressure may be accommodated by the embodiment.

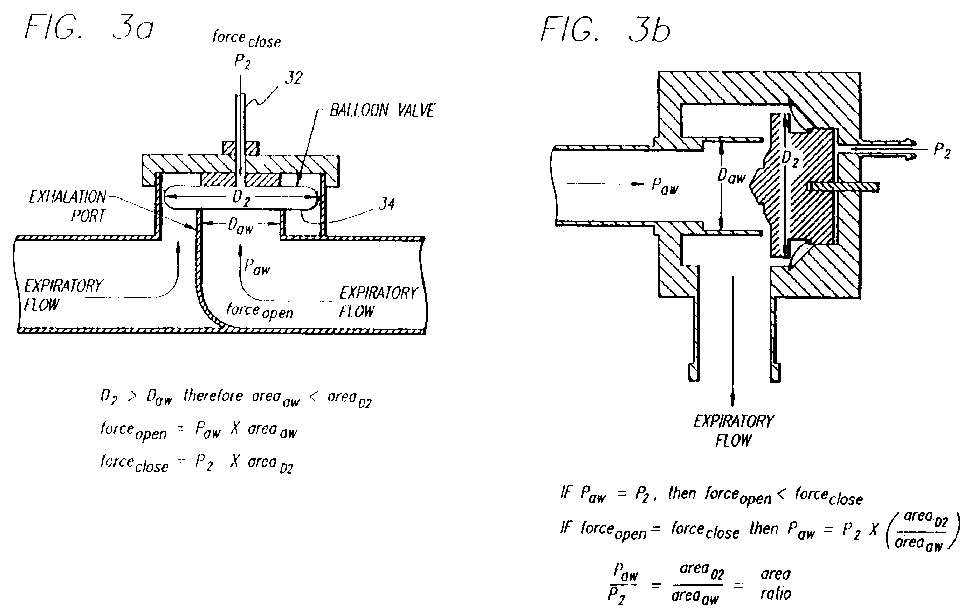

While a variety of relief valves may be used with the invention, one particular form of force balanced valve utilized as exhalation valves has been shown to be advantageous. Figs. 3a and 3b illustrates two types of such exhalation valves, which are of the balloon or diaphragm type. The balloon type valve illustrated in Fig. 3(a) incorporates a balloon 30 inflated by pressure from pilot pressure inlet 32, the expiratory flow will open the valve when the expiratory flow force on the valve exceeds the force created from the pilot pressure.

Similarly, the diaphragm valve of Fig. 3(b) is provided with pilot pressure inlet 32 operating on the cavity formed by the valve exterior body, the valve poppet and the area behind diaphragm 34, thus controlling expiratory flow similarly to balloon valve 3(a). While it has been shown that such valves are useful for the purposes of the relief valve, other valves which are electrically, mechanically or pneumatically driven, or a combination of these drive schemes, can be advantageously utilized for the relief valve.

From the foregoing, it should be clear that embodiments of the invention provide for an improved pneumatically driven, electronically controlled, pressure control system for a ventilator system for providing breathing gas to a patient at a desired flow rate and pressure level. More particularly, these embodiments allow prevention of overshoot of patient airway pressure over the target pressure set by a care provider by allowing for the incorporation of target pressure flow trajectories of more sophisticated ventilation strategies.

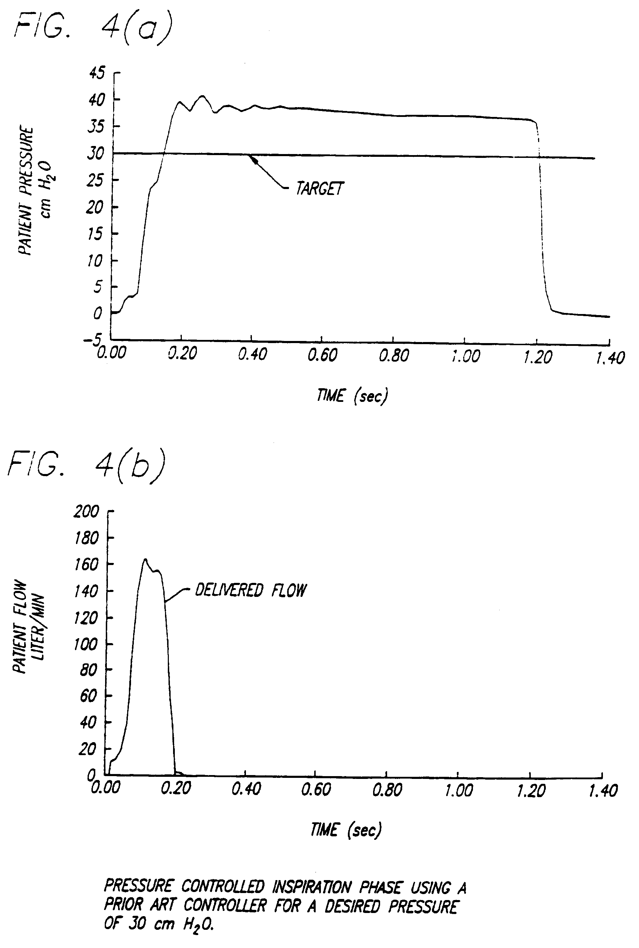

The use of the invention in combination with prior art ventilators has demonstrated substantial improvements in performance. Figs. 4(a) and 4(b) illustrate the pressure and flow time histories for a ventilator inspiration phase utilizing prior art controllers. More specifically, Fig. 4(a) illustrates the patient pressure in cm of H₂O at the patient wye for a commanded and desired pressure of 30 cm H₂O. As will be noted from this trace of pressure produced by prior art controllers, the patient airway pressure persists at a pressure on the order of 7 cm of H₂O above the desired pressure for a substantial portion of the breath after the beginning of inspiration. While this overshoot can be somewhat moderated if a slower initial inspiration rate is used, such a strategy results in less rapid filling of the lungs during the inspiration, and thus inferior ventilation. As illustrated in Fig. 4(b), when compared with Fig. 4(a), the patient overpressure persists long after the delivery of a breath is completed, an undesirable result of the use of prior art controllers.

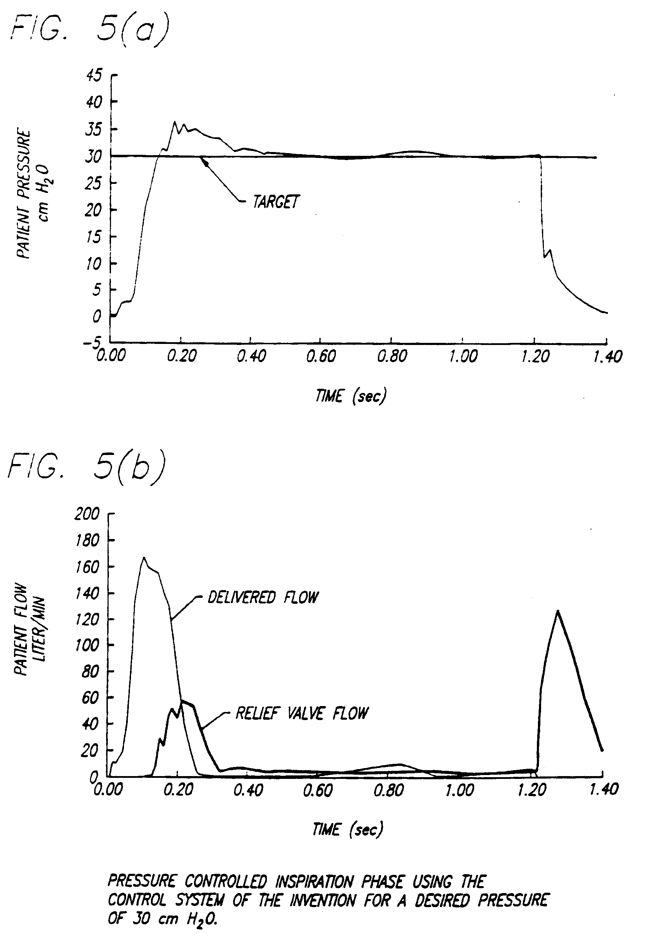

Figs. 5(a) and 5(b) illustrate the improved performance of a system utilizing the control system of the present invention. Referring to Fig. 5(a), a rapid rise time is utilized, as shown in Fig. 4(a), to facilitate rapid filling of the lungs of the patient once a breath is begun. Unlike the prior art controllers, however, the present invention is able to reduce the sustained overshoot of pressure after lung filling, and airway pressure can be maintained at the desired pressure of 30 cm H₂O. Referring to Fig. 5(b), the time history of flow rate flow to the patient is illustrated, as is the flow from the relief valve. It can be seen that the relatively modest flow rates from the relief valve near the early peaking of pressure can allow rapid return of patient airway pressure to the desired level. Additionally, as shown by the graph at the 1.2 second mark, the relief valve can be used to rapidly reduce pressure in the airway for the beginning of the expiration cycle. Thus, the use of the invention substantially reduces the sustained overpressure of the patent when compared to prior art controllers.

It should be recognized that although the invention has been described as providing individual, separate pressure, flow, and exhalation controllers, it is also possible that these functions can be performed by one or more microprocessors with appropriate inputs from the flow and pressure sensors provided, and outputs to the supply flow valve and pressure regulator mechanism.

It will be apparent from the foregoing that while particular forms of the invention have been illustrated and described, various modifications can be made without departing from the scope of the invention. Accordingly, it is not intended that the invention be limited, except as by the appended claims.

## Claims

1. A method of controlling airway pressure in a breathing gas circuit (6) of a ventilator for a patient during the inspiratory cycle of a breath, the breathing gas circuit (6) having a source of breathing gas, an airway flow path in fluid communication with a breathing attachment of the patient for supplying the breathing gas to the breathing attachment, a flow supply valve (14) for controlling the supply of breathing gas to the breathing attachment, and a relief valve (18) for controlling venting of breathing gas from the patient to the atmosphere, the method comprising the step of delivering a supply flow of breathing gas to the breathing attachment through the flow supply valve (14) at a predetermined rate of supply flow based upon a desired airway pressure and actual airway pressure, characterised in that the method further comprises the steps of determining a desired rate of outflow, measuring the actual rate of outflow through the relief valve (18), comparing the actual rate of outflow with the desired rate of outflow to determine an outflow error, generating a relief valve command signal derived from a signal representing the desired airway pressure and a signal representing the outflow error to control operation of the relief valve (18), and, during the inspiratory cycle, venting the breathing gas from the breathing attachment through the relief valve (18) in accordance with the relief valve command signal to achieve the desired airway pressure.

2. The method of Claim 1, further including the steps of measuring the actual rate of supply flow from the flow supply valve (14) to the breathing attachment, comparing the actual rate of supply flow with the predetermined rate of supply flow to determine a supply flow error, and generating a supply valve command signal based upon the supply flow error to control operation of the flow supply valve (14) to deliver the breathing gas at the predetermined rate of supply flow.

3. The method of Claim 1, further including the steps of measuring the actual airway pressure, comparing the actual airway pressure with an instantaneous desired airway pressure to determine an airway pressure error, and modifying the desired rate of flow based upon the airway pressure error.

4. A system for closed loop control of airway pressure in a breathing gas circuit (6) of a ventilator for a patient during the inspiratory cycle of a breath, the breathing gas circuit (6) having a source of breathing gas, and an airway flow path in fluid communication with a breathing attachment of the patient for supplying the breathing gas to the breathing attachment, the system comprising flow supply valve means (14) in said airway flow path for delivering a supply flow of breathing gas from the source of breathing gas to the breathing attachment at a desired rate of supply flow based upon a desired airway pressure and the actual airway pressure; and relief valve means (18) for the venting of breathing gas from the breathing attachment to the atmosphere during the inspiratory cycle, characterised in that the system further includes means for determining a desired rate of outflow, means (28) for measuring the actual rate of outflow through the relief valve means (18), means (26) for comparing the actual rate of outflow with the desired rate of outflow to determine an outflow error, and means (20, 22, 24) for generating a relief valve command signal derived from a signal representing the desired airway pressure and a signal representing the outflow error to control operation of the relief valve means (18) to vent the breathing gas from the breathing attachment through the relief valve means (18) during the inspiratory cycle to achieve the desired airway pressure.

5. The system of Claim 4, further including means (16) for measuring the actual rate of supply flow from the flow supply valve means (14) to the breathing attachment, means (10) for comparing the actual rate of supply flow with the desired rate of supply flow to determine a supply flow error, and means (12) for generating a supply valve command signal based upon the supply flow error to control operation of the flow supply valve means (14) to deliver the breathing gas at the desired rate of supply flow.

6. The system of Claim 4, further including means (4) for measuring the actual airway pressure, means (2) for comparing the actual airway pressure with the desired airway pressure to determine an airway pressure error, and means (8) for modifying the desired rate of flow based upon the airway pressure error.

7. An apparatus for the control of airway pressure in a ventilation respirator of a patient, the apparatus comprising means to set a desired target airway pressure; a pressure controller (8); a flow valve controller (12); a breathing gas flow valve (14); a sensor (16) for measuring the output flow of breathing gas from said breathing gas flow valve (14); a patient tubing airway system (6) in fluid communication with said flow valve (14) and the patient respiratory system; a pressure sensor (4) in fluid communication with said patient tubing airway system (6); first summing means (2) to create an error signal between the predetermined airway pressure and the signal from the pressure sensor (4), said error signal providing the signal to drive the pressure controller (8); second summing means (10) to determine the error between the output signal from the pressure controller (8) and the output signal from the patient airway flow sensor (16), said error signal providing the input to said flow valve controller (12); and a relief valve (18) in fluid communication with said patient tubing airway system (6), characterised in that the apparatus further comprises a relief valve flow sensor (28) in fluid communication with the output flow of said relief valve (18); a relief valve pressure controller (20) which outputs a command signal based upon a desired airway pressure; a third summing means (26) which outputs an error signal based upon the difference between a desired relief flow and the relief valve flow measured by the relief valve flow sensor (28); a relief valve flow controller (24) which outputs a relief valve command signal based upon the error signal from said third summing means (26); and fourth summing means (22) to output a relief valve command signal to said relief valve (18) to vent flow during the inspiratory cycle of a breath based upon the sum of the output signals of said relief valve pressure controller (20) and said relief valve flow controller (24) for controlling said relief valve (18) so as to achieve said desired target airway pressure.

## Patentansprüche

1. Verfahren zum Steuern/Regeln eines Luftwegdrucks in einem Atemgaskreis (6) eines Beatmungsgeräts für einen Patienten während des Einatmungszyklus eines Atemzugs, wobei der Atemgaskreis (6) eine Atemgasquelle, einen Luftwegströmungspfad in Fluidverbindung mit einem Atmungsvorsatz für den Patienten zur Zufuhr des Atemgases zu dem Atmungsvorsatz, ein Strömungszufuhrventil (14) zum Steuern der Zufuhr des Atemgases zu dem Atmungsvorsatz sowie ein Überdruckventil (18) zum Steuern der Atemgasabgabe vom Patienten zur Atmosphäre umfaßt, wobei das Verfahren den Schritt umfaßt, dem Atmungsvorsatz über das Strömungszufuhrventil (14) eine Zufuhrströmung des Atemgases mit einer vorbestimmten Zufuhrströmungsrate auf Grundlage eines gewünschten Luftwegdrucks und eines Ist-Luftwegdrucks zu liefern, dadurch gekennzeichnet, daß das Verfahren ferner die Schritte umfaßt: Bestimmen einer gewünschten Ausströmrate, Messen der Ist-Ausströmrate über das Überdruckventil (18), Vergleichen der Ist-Ausströmrate mit der gewünschten Ausströmrate, um einen Ausströmfehler zu ermitteln, Erzeugen eines Überdruckventilbefehlssignals, das aus einem den gewünschten Luftwegdruck repräsentierenden Signal und einem den Ausströmfehler repräsentierenden Signal hergeleitet wird, um den Betrieb des Überdruckventils (18) zu steuern, und - während des Einatmungszyklus - Abgeben des Atemgases aus dem Atmungsvorsatz über das Überdruckventil (18) nach Maßgabe des Überdruckventilbefehlssignals, um den gewünschten Luftwegdruck zu erreichen.

2. Verfahren nach Anspruch 1, ferner umfassend die Schritte: Messen der Ist-Zufuhrströmungsrate von dem Strömungszufuhrventil (14) zu dem Atmungsvorsatz, Vergleichen der Ist-Zufuhrströmungsrate mit der vorbestimmten Zufuhrströmungsrate, um einen Zufuhrströmungsfehler zu ermitteln, und Erzeugen eines Zufuhrventilbefehlssignals auf Grundlage des Zufuhrströmungsfehlers, um den Betrieb des Strömungszufuhrventils (14) so zu steuern, daß das Atemgas mit der vorbestimmten Zufuhrströmungsrate geliefert wird.

3. Verfahren nach Anspruch 1, ferner umfassend die Schritte: Messen des Ist-Luftwegdrucks, Vergleichen des Ist-Luftwegdrucks mit einem augenblicklichen gewünschten Luftwegdruck, um einen Luftwegdruckfehler zu ermitteln, und Modifizieren der gewünschten Strömungsrate auf Grundlage des Luftwegdruckfehlers.

4. System zum Regeln eines Luftwegdrucks in einem Atemgaskreis (6) eines Beatmungsgeräts für einen Patienten während des Einatmungszyklus eines Atemzugs, wobei der Atemgaskreis (6) eine Atemgasquelle und einen Luftwegströmungspfad in Fluidverbindung mit einem Atmungsvorsatz für den Patienten zur Zufuhr des Atemgases zu dem Atmungsvorsatz umfaßt, wobei das System ein Strömungszufuhrventilmittel (14) in dem Luftwegströmungspfad umfaßt, um dem Atmungsvorsatz von der Atemgasquelle eine Zufuhrströmung des Atemgases mit einer gewünschten Zufuhrströmungsrate auf Grundlage eines gewünschten Luftwegdrucks und des Ist-Luftwegdrucks zu liefern, sowie ein Überdruckventilmittel (18) umfaßt, um während des Einatmungszyklus Atemgas von dem Atmungsvorsatz zur Atmosphäre abzugeben, dadurch gekennzeichnet, daß das System ferner umfaßt: ein Mittel zum Bestimmen einer gewünschten Ausströmrate, ein Mittel (28) zum Messen der Ist-Ausströmrate über das Überdruckventilmittel (18), ein Mittel (26) zum Vergleichen der Ist-Ausströmrate mit der gewünschten Ausströmrate, um einen Ausströmfehler zu ermitteln, sowie ein Mittel (20, 22, 24) zum Erzeugen eines Überdruckventilbefehlssignals, das von einem den gewünschten Luftwegdruck repräsentierenden Signal und einem den Ausströmfehler repräsentierenden Signal hergeleitet wird, um den Betrieb des Überdruckventilmittels (18) zur Abgabe des Atemgases von dem Atmungsvorsatz über das Überdruckventilmittel (18) während des Einatmungszyklus so zu steuern, daß der gewünschte Luftwegdruck erreicht wird.

5. System nach Anspruch 4, ferner umfassend ein Mittel (16) zum Messen der Ist-Zufuhrströmungsrate von dem Strömungszufuhrventilmittel (14) zum Atmungsvorsatz, ein Mittel (10) zum Vergleichen der Ist-Zufuhrströmungsrate mit der gewünschten Zufuhrströmungsrate, um einen Zufuhrströmungsfehler zu ermitteln, und ein Mittel (12) zum Erzeugen eines Zufuhrventilbefehlssignals auf Grundlage des Zufuhrströmungsfehlers, um den Betrieb des Strömungszufuhrventilmittels (14) so zu steuern, daß das Atemgas mit der gewünschten Zufuhrströmungsrate geliefert wird.

6. System nach Anspruch 4, ferner umfassend ein Mittel (4) zum Messen des Ist-Luftwegdrucks, ein Mittel (2) zum Vergleichen des Ist-Luftwegdrucks mit dem gewünschten Luftwegdruck, um einen Luftwegdruckfehler zu ermitteln, und ein Mittel (8) zum Modifizieren der gewünschten Strömungsrate auf Grundlage des Luftwegdruckfehlers.

7. Vorrichtung zum Steuern/Regeln eines Luftwegdrucks in einem Beatmungsgerät für einen Patienten, wobei die Vorrichtung umfaßt: ein Mittel zum Festsetzen eines gewünschten Soll-Luftwegdrucks, ein Drucksteuerorgan (8), ein Strömungsventilsteuerorgan (12), ein Atemgasströmungsventil (14), einen Sensor (16) zum Messen der Ausgangsströmung des Atemgases von dem Atemgasströmungsventil (14), ein Patienten-Schlauchluftwegsystem (6) in Fluidverbindung mit dem Strömungsventil (14) und dem Patientenatmungssystem, einen Drucksensor (4) in Fluidverbindung mit dem Patienten-Schlauchluftwegsystem (6), ein erstes Summiermittel (2) zur Erzeugung eines Fehlersignals zwischen dem vorbestimmten Luftwegdruck und dem Signal von dem Drucksensor (4), wobei das Fehlersignal das Signal zur Ansteuerung des Drucksteuerorgans (8) bereitstellt, ein zweites Summiermittel (10) zur Bestimmung des Fehlers zwischen dem Ausgangssignal von dem Drucksteuerorgan (8) und dem Ausgangssignal von dem Patientenluftwegströmungssensor (16), wobei dieses Fehlersignal die Eingabe für das Strömungsventilsteuerorgan (12) bereitstellt, und ein Überdruckventil (18) in Fluidverbindung mit dem Patienten-Schlauchluftwegsystem (6), dadurch gekennzeichnet, daß die Vorrichtung ferner umfaßt: einen Überdruckventilströmungssensor (28) in Fluidverbindung mit der Ausgangsströmung des Überdruckventils (18), ein Überdruckventildrucksteuerorgan (20), welches ein Befehlssignal auf Grundlage eines gewünschten Luftwegdrucks ausgibt, ein drittes Summiermittel (26), welches ein Fehlersignal auf Grundlage der Differenz zwischen einer gewünschten Überdruckströmung und der von dem Überdruckventilströmungssensor (28) gemessenen Überdruckventilströmung ausgibt, ein Überdruckventilströmungssteuerorgan (24), welches ein Überdruckventilbefehlssignal auf Grundlage des Fehlersignals von dem dritten Summiermittel (26) ausgibt, und ein viertes Summiermittel (22) zur Ausgabe eines Überdruckventilbefehlssignals zu dem Überdruckventil (18), um während des Einatmungszyklus eines Atemzugs eine Strömung auf Grundlage der Summe der Ausgangssignale des Überdruckventildrucksteuerorgans (20) und des Überdruckventilströmungssteuerorgans (24) abzugeben, um das Überdruckventil (18) so zu steuern, daß der gewünschte Soll-Luftwegdruck erreicht wird.

## Revendications

1. Procédé de régulation de la pression des voies aériennes dans un circuit de gaz respiratoire (6) d'un respirateur destiné à un patient au cours du cycle inspiratoire d'une respiration, le circuit de gaz respiratoire (6) ayant une source de gaz respiratoire, un trajet de passage des voies aériennes en communication fluidique avec une connexion respiratoire du patient destinée à délivrer le gaz respiratoire à la connexion respiratoire, une vanne d'amenée (14) permettant de réguler l'amenée de gaz respiratoire dans la connexion respiratoire, et une soupape de sûreté (18) pour réguler l'évacuation du gaz respiratoire du patient à l'atmosphère, le procédé comprenant l'étape consistant à délivrer un flux d'amenée de gaz respiratoire à la connexion respiratoire par l'intermédiaire de la vanne d'amenée (14) à un débit d'amenée prédéterminé basé sur une pression souhaitée des voies aériennes et une pression réelle des voies aériennes, caractérisé en ce que le procédé comprend en outre les étapes consistant a déterminer un débit de sortie souhaité, à mesurer le débit de sortie réel à travers la soupape de sûreté (18), à comparer le débit de sortie réel avec le débit de sortie souhaité pour déterminer une erreur de sortie, à générer un signal de commande de soupape de sûreté dérivé d'un signal représentant la pression souhaitée des voies aériennes et un signal représentant l'erreur de sortie pour commander le fonctionnement de la soupape de sûreté (18) et, pendant le cycle inspiratoire, à évacuer le gaz respiratoire de la connexion respiratoire par l'intermédiaire de la soupape de sûreté (18) en fonction du signal de commande de soupape de sûreté pour atteindre la pression souhaitée des voies aériennes.

2. Procédé selon la revendication 1, comprenant en outre les étapes consistant à mesurer le débit d'amenée réel depuis la vanne d'amenée (14) jusqu'à la connexion respiratoire, à comparer le débit d'amenée réel avec le débit d'amenée prédéterminé pour déterminer une erreur d'amenée, et à générer un signal de commande de vanne d'amenée en fonction de l'erreur d'amenée pour commander le fonctionnement de la vanne d'amenée (14) afin de délivrer le gaz respiratoire prédéterminé au débit d'amenée.

3. Procédé selon la revendication 1, comprenant en outre les étapes consistant à mesurer la pression réelle des voies aériennes, à comparer la pression réelle des voies aériennes avec une pression instantanée souhaitée des voies aériennes pour déterminer une erreur de pression des voies aériennes, et à modifier le débit souhaité en fonction de l'erreur de pression des voies aériennes.

4. Système pour la régulation en boucle fermée de la pression des voies aériennes dans un circuit de gaz respiratoire (6) d'un respirateur destiné à un patient pendant le cycle inspiratoire d'une respiration, le circuit de gaz respiratoire (6) comprenant une source de gaz respiratoire et un trajet de passage des voies aériennes en communication fluidique avec une connexion respiratoire du patient destinée à délivrer le gaz respiratoire à la connexion respiratoire, le système comprenant des moyens formant vanne d'amenée (14) dans ledit trajet de passage des voies aériennes pour délivrer un flux d'amenée de gaz respiratoire de la source de gaz respiratoire à la connexion respiratoire à un débit d'amenée souhaité à partir d'une pression souhaitée des voies aériennes et la pression réelle des voies aériennes ; et des moyens formant soupape de sûreté (18) destinés à l'évacuation du gaz respiratoire de la connexion respiratoire à l'atmosphère pendant le cycle inspiratoire, caractérisé en ce que le système comprend en outre des moyens pour déterminer un débit de sortie souhaité, des moyens (28) pour mesurer le débit de sortie réel à travers les moyens formant soupape de sûreté (18), des moyens (26) pour comparer le débit de sortie réel avec le débit de sortie souhaité pour déterminer une erreur de sortie, et des moyens (20, 22, 24) pour générer un signal de commande de soupape de sûreté dérivé d'un signal représentant la pression souhaitée des voies aériennes et un signal représentant l'erreur de sortie pour réguler le fonctionnement des moyens formant soupape de sûreté (18) pour évacuer le gaz respiratoire depuis la connexion respiratoire à travers les moyens formant soupape de sûreté (18) pendant le cycle inspiratoire pour atteindre la pression souhaitée des voies aériennes.

5. Système selon la revendication 4, comprenant en outre des moyens (16) pour mesurer le débit d'amenée réel depuis les moyens formant vanne d'amenée (14) jusqu'à la connexion respiratoire, des moyens (10) pour comparer le débit d'amenée réel avec le débit d'amenée souhaité pour déterminer une erreur d'amenée, et des moyens (12) pour générer un signal de commande de vanne d'amenée en fonction de l'erreur d'amenée pour réguler le fonctionnement des moyens formant vanne d'amenée (14) pour délivrer le gaz respiratoire au débit d'amenée souhaité.

6. Système selon la revendication 4, comprenant en outre des moyens (4) pour mesurer la pression réelle des voies aériennes, des moyens (2) pour comparer la pression réelle des voies aériennes avec la pression souhaitée des voies aériennes afin de déterminer une erreur de pression des voies aériennes, et des moyens (8) pour modifier le débit souhaité en fonction de l'erreur de pression des voies aériennes.

7. Appareil de régulation de la pression des voies aériennes dans un respirateur destiné à un patient, l'appareil comprenant des moyens pour définir une pression visée souhaitée ; un régulateur de pression (8) ; un régulateur de débit (12) ; un régulateur de gaz respiratoire (14) ; un capteur (16) pour mesurer le débit de sortie de gaz respiratoire depuis ledit régulateur de gaz respiratoire (14) ; un système de tubes d'aération de patient (6) en communication fluidique avec ledit régulateur (14) et le système respiratoire du patient ; un capteur de pression (4) en communication fluidique avec ledit système de tubes d'aération du patient (6) ; des premiers moyens d'addition (2) pour créer un signal d'erreur entre la pression prédéterminée des voies aériennes et le signal provenant du capteur de pression (4), ledit signal d'erreur délivrant le signal pour entraîner le régulateur de pression (8) ; des deuxièmes moyens d'addition (10) pour déterminer l'erreur entre le signal de sortie provenant du régulateur de pression (8) et le signal de sortie provenant du capteur de débit (16) des voies aériennes du patient, ledit signal d'erreur fournissant l'entrée audit régulateur de débit (12) ; et une soupape de sûreté (18) en communication fluidique avec ledit système de tubes d'aération de patient (6), caractérisé en ce que l'appareil comprend en outre un capteur de débit de soupape de sûreté (28) en communication fluidique avec le débit de sortie de ladite soupape de sûreté (18) ; un régulateur de pression de soupape de sûreté (20) qui délivre un signal de commande basé sur une pression souhaitée des voies aériennes ; des troisièmes moyens d'addition (26) qui délivrent un signal d'erreur basé sur la différence entre un débit de sûreté souhaité et le débit de la soupape de sûreté mesuré par le capteur de débit de soupape de sûreté (28) ; un régulateur de débit de soupape de sûreté (24) qui délivre un signal de commande de soupape de sûreté basé sur le signal d'erreur provenant desdits troisièmes moyens d'addition (26) ; et des quatrièmes moyens d'addition (22) pour délivrer un signal de commande de soupape de sûreté à ladite soupape de sûreté (18) pour évacuer le débit pendant le cycle inspiratoire d'une respiration, basé sur la somme des signaux de sortie dudit régulateur de pression de soupape de sûreté (20) et dudit régulateur de débit de soupape de sûreté (24) afin de commander ladite soupape de sûreté (18) pour atteindre ladite pression visée des voies aériennes.
